# EUROPEAN PATENT APPLICATION

(11) **EP 4 763 841 A1**
(43) Date of publication of application: **24.06.2026**
(21) Application number: 24853554.4
(22) Date of filing: 30.07.2024
(51) Int. Cl.: C07D 403/12, C07D 403/10, C07D 237/14, C07D 237/16, A61K 31/501, A61K 31/53, A61P 5/16, A61P 3/06, A61P 9/00, A61P 9/10, A61P 35/00

(54) **PYRIDAZINONE COMPOUND AND USE THEREOF**

(30) Priority: 16.08.2023 CN 202311033411; 06.11.2023 CN 202311458253; 15.01.2024 CN 202410051370
(71) Applicant: Xi'an Xintong Pharmaceutical Research Co., Ltd., Xi'an, Shaanxi 710077 (CN)
(72) Inventor: QIN, Long, Shaanxi 710077 (CN); WANG, Mailing, Shaanxi 710077 (CN); ZHANG, Chi, Shaanxi 710077 (CN); GUO, Jin, Shaanxi 710077 (CN); ZHANG, Lan, Shaanxi 710077 (CN); FAN, Siyuan, Shaanxi 710077 (CN); QIANG, Wenzhou, Shaanxi 710077 (CN); WANG, Yaowen, Shaanxi 710077 (CN); LIU, Miao, Shaanxi 710077 (CN); GAO, Zhongqiang, Shaanxi 710077 (CN); WANG, Yuan, Shaanxi 710077 (CN); GUO, Weibo, Shaanxi 710077 (CN); ZHANG, Qiang, Shaanxi 710077 (CN)
(74) Representative: Murgitroyd & Company
(86) International application number: PCT/CN2024/108330
(87) International publication number: WO 2025/036142

(57) **Abstract**

The present invention provides a pyridazinone compound, or a stereoisomer, tautomer or pharmaceutically acceptable salt thereof, and also provides a related preparation method for the compound and use thereof, a pharmaceutical composition including the compound, and a method for treating related metabolic diseases. The compound of the present invention exhibits excellent thyroid hormone receptor agonistic effects, and has broad application prospects in the field of metabolic disease treatment.

## Description

### TECHNICAL FIELD

The present invention relates to a pyridazinone compound, a preparation method therefor, and use of the compound in a medicament for the treatment of diseases.

### BACKGROUND

Thyroid hormones are critical for normal growth and development and for maintaining metabolic homeostasis. Circulating levels of thyroid hormones are tightly regulated by a feedback mechanism in a hypothalamic-pituitary-thyroid (HPT) axis. Thyroid dysfunction that leads to hypothyroidism or hyperthyroidism clearly demonstrates that thyroid hormones have profound effects on the cardiac function, a body weight, metabolism, a metabolic rate, the body temperature, cholesterol, bones, muscles, and behaviors.

The biological activity of thyroid hormones is mediated by thyroid hormone receptors (THRs). Thyroid hormone receptor subtypes may differ in the contribution to special physiological responses. THRβ plays an important role in regulating thyroid stimulating hormone and regulating the effects of thyroid hormones in the liver. The development of thyroid analogs that avoid the adverse effects of hyperthyroidism and hypothyroidism while maintaining the beneficial effects of thyroid hormones will open a new way to treat patients with metabolic diseases such as obesity, hyperlipidemia, hypercholesterolemia, diabetes and other conditions such as hepatic steatosis and non-alcoholic steatohepatitis (NASH), atherosclerosis, cardiovascular diseases, hypothyroidism, thyroid cancer, thyroid diseases, and related conditions and diseases.

In recent years, a series of THRβ agonists have been developed, for example, Resmetirom has the effects of reducing liver fat accumulation and inhibiting liver fibroplasia. A structural formula of Resmetirom is as follows:

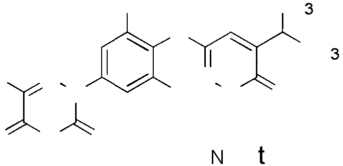

However, on the basis that a structure-activity relationship of pharmaceutical compounds is not obvious, how to develop compounds with better efficacy, better selectivity and better pharmacokinetic properties is still an arduous challenge. After unsuccessful research on deuteration of Resmetirom, the inventors surprisingly found that deuteration of certain Resmetirom analogs themselves and at certain positions thereof has greater potential, and the compounds thus developed exhibit excellent THRβ agonism and selectivity, and exhibit better effects of reducing liver fat accumulation and inhibiting liver fibroplasia than Resmetirom.

### SUMMARY

The present invention provides a compound of a formula (X), or a stereoisomer, tautomer, or pharmaceutically acceptable salt thereof, 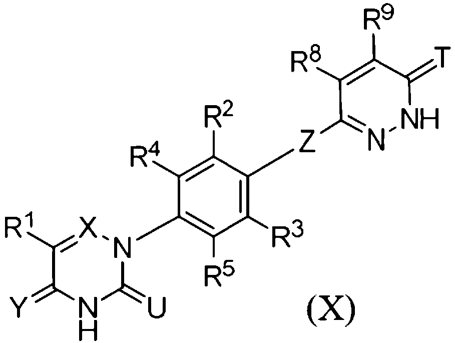 wherein X is a nitrogen atom or a carbon atom;
Y, U, and T are each independently a nitrogen atom, an oxygen atom, or a sulfur atom; Z is -NH-, -O-, -S-, or -CH₂-;
R¹ is selected from hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, alkenyl, alkynyl, cyano, formyl, or alkoxycarbonyl; and
R², R³, R⁴, R⁵, R⁸, and R⁹ are each independently hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, phosphocarbonyl, substituted silyl, alkenyl, or alkynyl.

The present invention provides a compound of a formula (I), or a stereoisomer, tautomer, or pharmaceutically acceptable salt thereof, 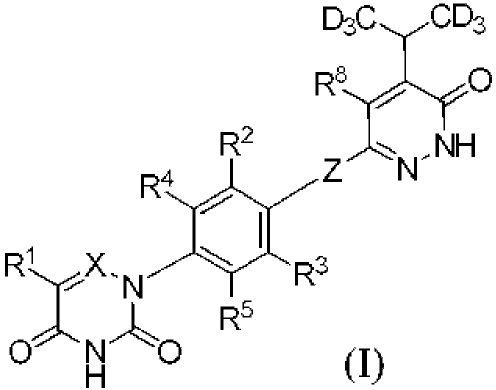
wherein X is a nitrogen atom or a carbon atom;
Z is -NH-, -O-, -S-, or -CH₂-;
R¹ is selected from hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, alkenyl, alkynyl, cyano, formyl, or alkoxycarbonyl; and
R², R³, R⁴, R⁵, and R⁸ are each independently hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, phosphocarbonyl, substituted silyl, alkenyl, or alkynyl.

The present invention provides a compound of a formula (II), or a stereoisomer, tautomer, or pharmaceutically acceptable salt thereof, 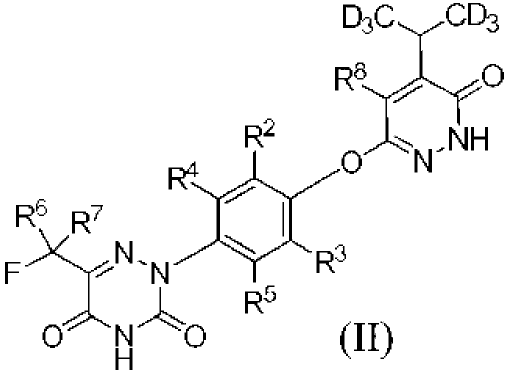 wherein R², R³, R⁴, R⁵, R⁶, R⁷, and R⁸ are each independently hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, phosphocarbonyl, substituted silyl, alkenyl, or alkynyl.

Preferably, the present invention provides the following compounds, or stereoisomers, tautomers or pharmaceutically acceptable salts thereof,

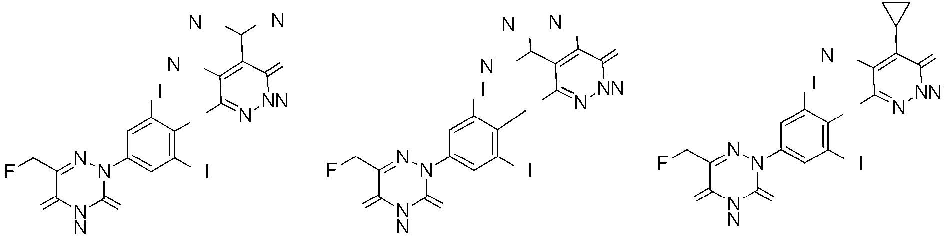

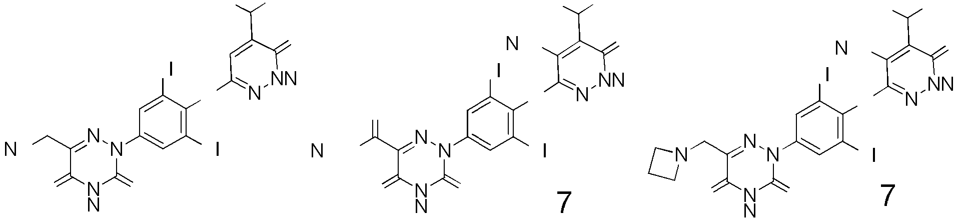

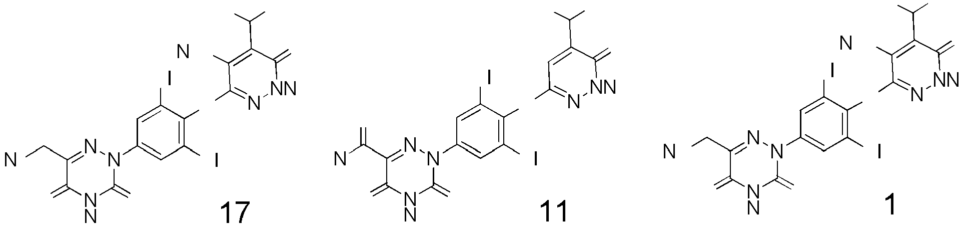

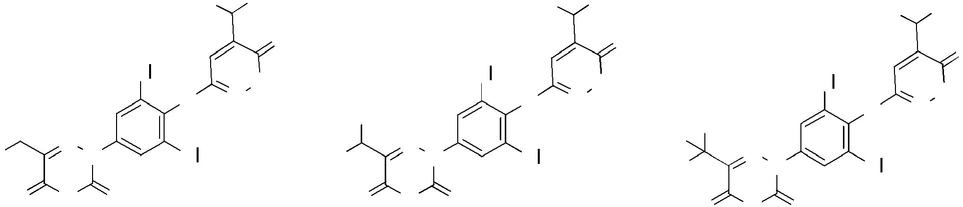

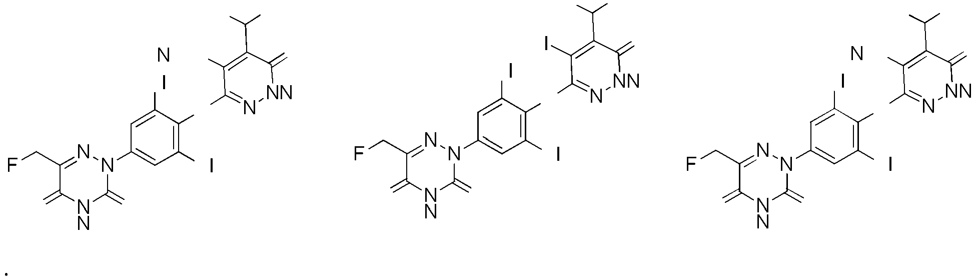

The present invention provides a pharmaceutical composition, including a therapeutically effective amount of any one compound, or the stereoisomer, tautomer or pharmaceutically acceptable salt thereof according to the present invention, and a pharmaceutically acceptable carrier.

The present invention provides use of the compound or the pharmaceutically acceptable salt thereof according to the present invention, or the pharmaceutical composition according to the invention in the manufacture of a medicament for thyroid hormone receptor (THR)-related diseases. Additionally, the present invention provides a method for treating a disease with a thyroid hormone receptor agonist, including administering to a subject in need thereof a therapeutically effective amount of the compound or the stereoisomer, tautomer or pharmaceutically acceptable salt thereof according to the present invention.

Preferably, wherein a disease that can be treated by the thyroid hormone receptor agonist is a metabolic disease, more preferably obesity, hyperlipidemia, hypercholesterolemia, diabetes, non-alcoholic steatohepatitis (NASH), atherosclerosis, cardiovascular disease, hypothyroidism or thyroid cancer.

All technical and scientific terms used in this specification have the meanings commonly understood by those of ordinary skill in the art.

| Term | Meaning herein |
|---|---|
| Hydrogen | -H |
| Deuterium | -D |
| Halogen | -F, -Cl, -Br and -I |
| Fluorine | -F |
| Chlorine | -Cl |
| Bromine | -Br |
| Iodine | -I |
| Cyano | -CN |
| Amino | -NH₂ |
| Hydroxyl | -OH |
| Phosphocarbonyl | 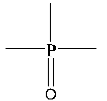 |
| Substituted silyl | 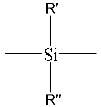 |

The term "alkyl" herein refers to a saturated aliphatic hydrocarbonyl group with 1 to 10 carbon atoms, and the alkyl includes linear hydrocarbonyl and branched hydrocarbonyl. Non-limiting examples of alkyl include methyl, ethyl, n-propyl, isopropyl, n-butyl, isobutyl, sec-butyl, tert-butyl, n-pentyl, neopentyl, n-hexyl, and the like. The alkyl described herein may be optionally substituted by one or more of deuterium, fluorine, chlorine, bromine, iodine, cyano, nitro, hydroxyl, carboxyl, amino, alkyl, alkoxy, acyl, acyloxy, oxo, an amide group, an ester group, an amine group, cycloalkyl, cycloalkenyl, heterocycloalkyl, alkenyl, alkenyloxy, alkynyl, cycloalkoxy, heterocycloalkoxy, aryloxy, heteroaryloxy, aryl or heteroaryl.

The term "aryl" herein refers to a 6- to 10-membered all-carbon monocyclic or fused polycyclic (i.e., rings which share a pair of adjacent carbon atoms) group, or a polycyclic (i.e., rings with a pair of adjacent carbon atoms) group having a conjugated π-electron system. The aryl may be covalently bonded to the defined chemical structure at any carbon atom that produces a stable structure. The aryl described herein may be optionally substituted by one or more of fluorine, chlorine, bromine, iodine, cyano, nitro, hydroxyl, carboxyl, amino, alkyl, alkoxy, acyl, an amide group, an ester group, an amine group, sulfonyl, sulfinyl, cycloalkyl, cycloalkenyl, heterocycloalkyl, alkenyl, alkynyl, and cycloalkoxy.

The term "heterocyclyl" refers to a ring system containing a nitrogen atom or containing an oxygen atom, and the ring system can be formed by "fusing" aromatic and non-aromatic ring systems, or linked to other ring systems through a "spiro carbon atom", such as the following structures: 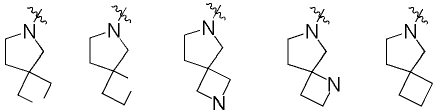 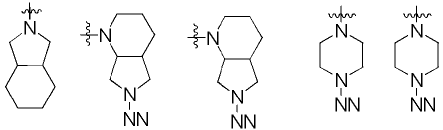 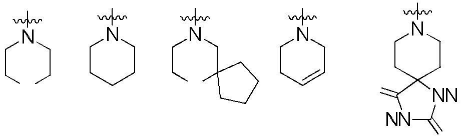 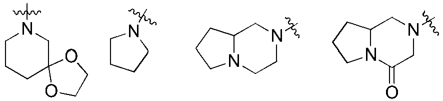 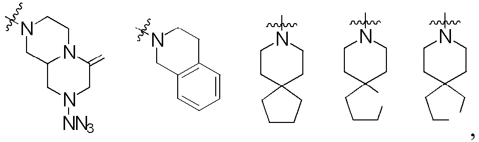 etc.

The term "heteroaryl" herein refers to an aromatic group consisting of 5 to 10 atoms and containing at least one heteroatom selected from N, O or S. The heteroaryl may have a single ring (non-limiting examples include furan, thiophene, imidazole, pyrazole, pyridine, pyrazine, oxazole, thiazole, etc.) or a plurality of fused rings (non-limiting examples include benzothiophene, benzofuran, indole, isoindole, etc.), wherein the fused ring may or may not be an aromatic group containing a heteroatom, provided that the connection occurs through an atom of an aromatic heteroaryl group. The heteroaryl described herein may be optionally substituted by one or more of fluorine, chlorine, bromine, iodine, cyano, nitro, hydroxyl, amino, alkyl, alkoxy, acyl, acyloxy, an amide group, an ester group, an amine group, sulfonyl, sulfinyl, cycloalkyl, cycloalkenyl, heterocycloalkyl, alkenyl, alkynyl, and cycloalkoxy.

The term "alkenyl" herein refers to an alkenyl group having 2 to 8 carbon atoms and having at least one site of alkenyl unsaturation. Non-limiting examples of the alkenyl group include ethenyl, propenyl, allyl, isopropenyl, butenyl, isobutenyl, and the like. The alkenyl described herein may be optionally substituted by one or more of deuterium, fluorine, chlorine, bromine, iodine, cyano, nitro, hydroxyl, carboxyl, amino, alkyl, alkoxy, acyl, an amide group, an ester group, an amine group, sulfonyl, sulfinyl, cycloalkyl, cycloalkenyl, heterocycloalkyl, cycloalkoxy, thiol, alkylthiol, deuteroalkylthiol, sulfonyl, sulfinyl, silyl, phosphonyl, deuteroalkyl, heterocycloalkyl, aryl, heteroaryl, alkynyl, alkenyl, and aralkyl.

The term "alkynyl" herein refers to alkyl in which two adjacent carbon atoms are connected by a triple bond, wherein the alkyl is as defined herein. Alkynyl refers to an unsaturated alkyl consisting of at least two carbon atoms and at least one carbon-carbon triple bond as defined above, such as ethynyl, 1-propynyl, 2-propynyl, 1-, 2- or 3-butynyl, and the like. Alkynyl may be substituted or unsubstituted, and when the alkenyl is substituted, substituents are preferably one or more of the following groups, independently selected from deuterium, fluorine, chlorine, bromine, iodine, cyano, nitro, hydroxyl, carboxyl, amino, alkyl, alkoxy, acyl, an amide group, an ester group, an amine group, sulfonyl, sulfinyl, cycloalkyl, cycloalkenyl, heterocycloalkyl, cycloalkoxy, thiol, alkylthiol, deuteroalkylthiol, sulfonyl, sulfinyl, silyl, phosphonyl, deuteroalkyl, heterocycloalkyl, aryl, heteroaryl, alkynyl, alkenyl, and aralkyl.

The term "pharmaceutically acceptable carrier" herein refers to a non-toxic solid, semi-solid or liquid filler, diluent, adjuvant, encapsulating material or other formulation auxiliaries. The carrier used may be adapted to a respective administration form, and carriers known to those skilled in the art may be formulated into injections, lyophilized powders (for injection), sprays, oral solutions, oral suspensions, tablets, capsules, enteric-coated tablets, pills, powders, granules, sustained-release or delayed-release formulations, etc.

The terms "prevention" and "treatment" herein have the meanings conventionally understood by those skilled in the art, i.e., "prevention" refers to the administration of a pharmaceutical composition or active compound prior to the onset of a disease or appearance of symptoms to prevent, delay and/or alleviate the onset or the appearance of symptoms of the corresponding condition; and "treatment" refers to the administration of a pharmaceutical composition or active compound at the time of or after the onset of a disease, or at the time of or after the appearance of symptoms to eliminate the corresponding condition, reduce the severity of the corresponding symptoms, or delay the development of the corresponding condition.

The term "subject" herein preferably refers to a mammal, particularly a human or an experimental animal (such as a mouse, rat or rabbit, etc.).

The term "effective amount" herein refers to an appropriate dose of a drug or treatment method required to produce a desired effect. A therapeutically effective amount refers to a dose of a drug capable of treating a disease or condition, and a prophylactically effective amount refers to a dose of a drug for a measure capable of preventing the occurrence of a disease or reducing the risk of a disease. The effective amount can be determined by those skilled in the art depending on specific factors, such as the age, body weight, severity of the condition, and the like, of the subject.

### List of abbreviations for other nouns

| English abbreviation | Full English name |
|---|---|
| NASH | Non-alcoholic steatohepatitis |
| NAFLD | Non-alcoholic fatty liver disease |
| I.G. | Intragastrical administration. |
| Q.D. | Quaque Die |
| ALT | Alanine aminotransferase |
| AST | Aspartate aminotransferase |
| TC | Total Cholesterol |
| TG | Triglyceride |
| SEM | Standard error of mean |
| CCL4 | Carbon tetrachloride |
| HFD | High-Fat Diet |

The beneficial effects of the present invention include:
(1) the compound of the present invention has a better agonistic effect on THRβ, and its agonistic activity on THRβ is significantly better than that of Resmetirom; on the other hand, compounds 6, 16, and 17 have weak agonistic activity on THRα, with little agonistic activity, indicating that the compounds 6, 16, and 17 have selective agonistic effects on THRβ, with significant safety advantages.
(2) The elimination phase half-life of the compound 6 (T_{1/2}=7.80 h) is significantly longer than that of Resmetirom (T_{1/2}=2.98 h) in the same study animal system at the same study dose.
(3) The compound 6 of the present invention has a significant liver distribution advantage over Resmetirom and a compound X1.
(4) The compound 6 shows better effects of reducing liver fat accumulation and inhibiting liver fibroplasia under the same experimental conditions than Resmetirom.

### BRIEF DESCRIPTION OF THE DRAWINGS

FIG. 1: Comparison of chromatograms after incubation of a compound 16 and a compound 6 with human liver microsome
FIG. 2: Histograms showing the concentration of compounds in blood, liver, kidney and heart at 1 h and 4 h after gavage in SD rats
FIG. 3: Dosing regimen of the efficacy test experiment in an NASH model of B-DIO mice
FIG. 4: Results of body weight determination of mice after administration in the efficacy test experiment in the NASH model
FIG. 5: Blood was collected from mice in groups G1-G6 on day 26 to determine the level of triglyceride
FIG. 6: Blood was collected from mice in the groups G1-G6 on day 26 to determine the level of transaminase
FIG. 7: Livers of mice in the groups G1-G6 were collected on day 26 to determine the levels of total cholesterol and triglyceride
FIG. 8: Livers of mice in the groups G1-G6 were collected on day 26, and frozen sections were embedded and stained with Oil Red O to determine the degree of fat changes in livers of the mice
FIG. 9: Livers of mice in the groups G1-G6 groups were collected on day 26, and after fixation and section making, Sirius red staining was performed to determine the degree of liver fibrosis in the mice

### DETAILED DESCRIPTION

Hereinafter, the present invention is further illustrated by the examples, but the present invention is not limited thereto. Throughout the present invention, various examples of a compound and a method of the present invention are mentioned herein. The present invention is not limited to these examples, and the following examples are merely intended to provide methods of practicing the present invention and do not limit the scope of the present invention in any way.

The compounds provided in the present invention can be prepared by standard synthetic methods well known in the art, and general methods for preparing the compounds of the invention are provided in the description. Starting materials are generally commercially available or prepared by methods well known to those skilled in the art.

A scheme is as follows:

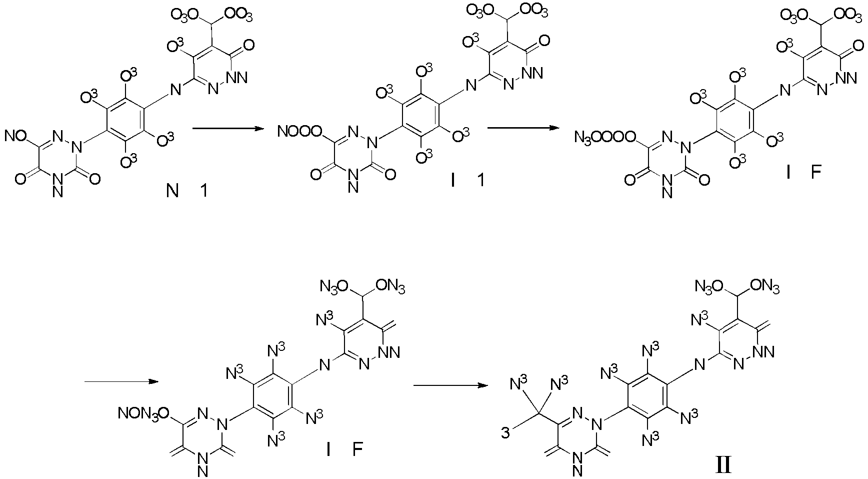

Cyano hydrolysis is performed by using SM-1 as a starting material to obtain a compound (IM-1), an esterification reaction is carried out to obtain a compound (IM-2), reduction is performed to obtain a compound (IM-3), and a functional group conversion reaction is carried out to obtain a compound (II).

The compounds of the present invention and corresponding preparation methods are further explained and exemplified below by the Examples and preparations. It will be appreciated that although typical or preferred reaction conditions are given in the specific examples, other reaction conditions may also be used by those skilled in the art. Optimum reaction conditions may vary depending on a particular reaction substrate or solvent used, but such conditions can be determined by those skilled in the art by routine optimization.

### Preparation of intermediates

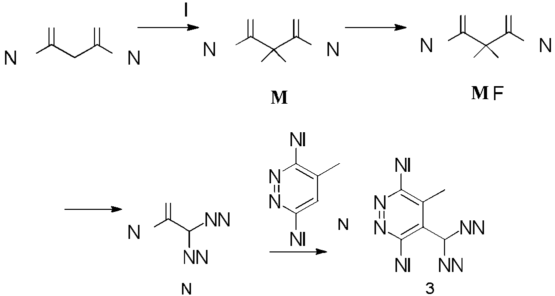

### First step:

Diethyl malonate (40.0 g) and absolute ethanol (320 mL) were added to a reaction flask, stirring was performed at ambient temperature until a clear solution was obtained, and sodium ethoxide (50.6 g) was added, and stirring was performed at ambient temperature for 0.5 h after the addition was complete. To the above solution was added deuteroiodomethane (80.0 g) in portions while controlling the temperature to be 20-30°C. A reaction was carried out for 2 h while maintaining at this temperature after the addition was complete. To the resulting reaction solution was added 200mL of water and 100mL of ethyl acetate, and stirring and liquid separation were performed. An aqueous layer was further extracted with 100mL of ethyl acetate. Ethyl acetate layers were mixed, sequentially washed with 200mL of a saturated aqueous sodium chloride solution, dried over anhydrous sodium sulfate, and concentrated under reduced pressure to dryness to give 25.4 g of a red-brown oil. m/z: 195.20 (M+1).

### Second step:

IM-1 (25.4 g), potassium hydroxide (18.5 g) and 125 mL of water were sequentially added to a reaction flask and heated to 70°C, and a reaction was carried out for 1.0 h. The resulting reaction solution was cooled to room temperature, adjusted with concentrated HCl to a pH of 2, and concentrated under reduced pressure, and water removal was performed once with 100 mL of methanol. To the above concentrate were added 250 mL of methanol and 25 g of anhydrous sodium sulfate, stirring was performed at ambient temperature for 0.5 h, filtration was performed, and a filtrate was concentrated under reduced pressure to dryness to give 19.8 g of a pale yellow solid. m/z: 139.10 (M+1), 137.10 (M-1).

### Third step:

IM-2 was heated to 185°C, and a reaction was carried out for 4 h. After the resulting reaction solution was cooled to room temperature, 80 mL of water and 80 mL of dichloromethane were added to the reaction solution, and stirring and liquid separation were performed. An aqueous layer was further extracted with 70 mL of dichloromethane. Dichloromethane layers were mixed, sequentially washed with 100 mL of saturated sodium chloride, dried over anhydrous sodium sulfate, and concentrated under reduced pressure to dryness to give 7.9 g of a red-brown oil.

### Fourth step:

IM-4 (4.1 g), IM-3 (2.8 g), silver nitrate (2.15 g), and sulfuric acid (12.3 g) were added to 40 mL of water, ammonium thiosulfate (21.8 g) was added after heating to 40°C, heating was performed to 70°C after addition was complete, and a reaction was carried out for 1 h. After the resulting reaction solution was cooled to ambient temperature, filtration was performed, and a filter cake was oven-dried to give 7.35 g of a grey solid. m/z: 211.05 (M+1).

### Preparation of compounds

### Example 1:

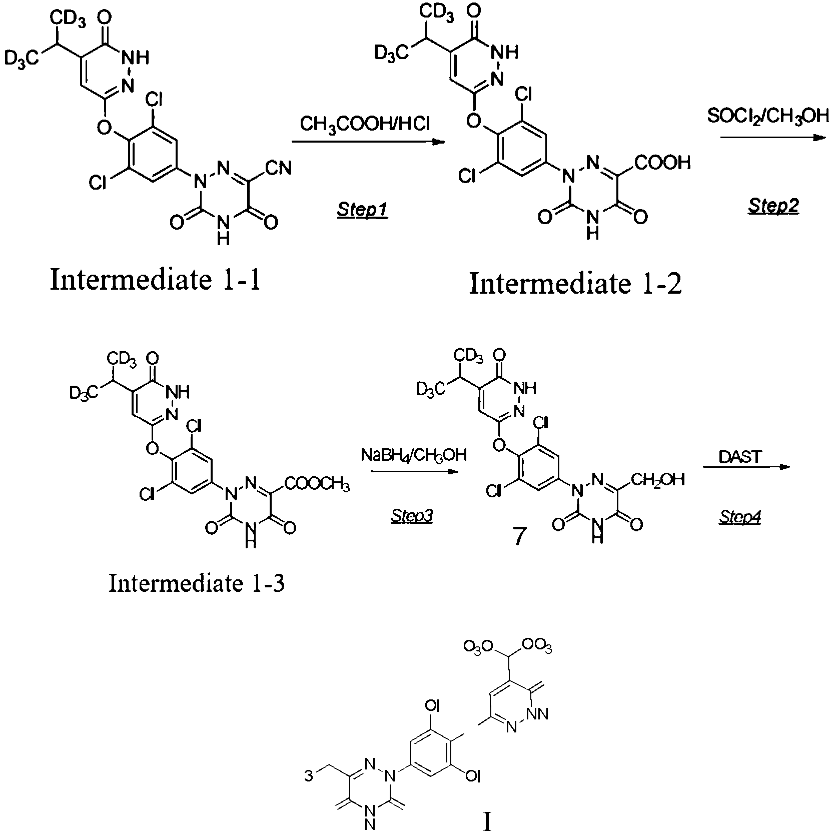

### First step:

An intermediate 1-1 (3 g) (prepared by the method in the document WO2019144835A1) was dissolved in ethyl acetate (100 mL), concentrated hydrochloric acid (20 mL) was added with stirring, heating was performed to 120°C after the addition was complete, and a reaction was carried out for 6 h. After completion of the reaction, the resulting reaction solution was cooled to room temperature, H₂O (200 mL) was added dropwise to the reaction solution, stirring was performed at room temperature for 1 h after the dropwise addition was complete, filtration was performed, a filter cake was rinsed with H₂O (20 mL×2), and a solid was collected and dried at 45°C to give 2.0 g of an earthy yellow solid.

### Second step:

An intermediate 1-2 (2 g) was dissolved in methanol (100 mL), and SOCl₂ (10 mL) was added dropwise under an ice bath. After the dropwise addition was complete, heating was performed to 100°C, and a reaction was carried out for 5 h. After completion of the reaction, the resulting reaction solution was concentrated under reduced pressure, a residue was slurried with ethyl acetate, filtration was performed, a filter cake was rinsed with ethyl acetate, and a solid was collected and dried at 45°C to give 2.3 g of a yellow solid.

### Third step:

An intermediate 1-3 was dissolved in tetrahydrofuran (40 mL), NaBH₄ (0.82 g) was added, methanol (4 mL) was slowly added dropwise after the addition was complete to generate a large number of bubbles, and a reaction was carried out under stirring for 1 h after the dropwise addition was complete. After completion of the reaction, the reaction was quenched with ice water, and the reaction solution was adjusted to a pH of about 6 with 1 M HCl, and extracted with ethyl acetate (20 mL×2), organic phases were mixed, washed with saturated NaCl (20 mL), dried over anhydrous sodium sulfate, filtered, and concentrated, and a residue was allowed to pass through a column to give 1.6 g of a light yellow solid. ¹H-NMR (400 MHz, DMSO-d₆) δ: 12.44 (s, 1H), 12.21 (s, 1H), 7.86 (s, 2H), 7.44 (d, 1H, J=0.8Hz), 5.31-5.27 (t, 1H, J=12.4&6.4Hz), 4.41 (d, 2H, J=5.6Hz), 3.10-3.00 (s, 1H).

### Fourth step:

A compound 7 (100 mg) was dissolved in dichloromethane (20 mL), diethylaminosulfur trifluoride (DAST) (8 drops) was added dropwise under an ice bath, stirring was performed under the ice bath for 30 min after the dropwise addition was complete. After completion of the reaction, the reaction solution was slowly added dropwise into ice water, liquid separation was performed, an organic phase was temporarily stored, an aqueous phase was extracted with dichloromethane (15 mL), organic phases were mixed, washed with saturated NaCl (20 mL), dried over anhydrous sodium sulfate, filtered, and concentrated, and a residue was slurried with ethyl acetate to give 20 mg of a white solid. m/z: 448.00 (M+H); ¹H-NMR (400 MHz, DMSO-d₆) δ: 12.67 (s, 1H), 12.22 (s, 1H), 7.82 (s, 2H), 7.44 (d, 1H, J=0.8Hz), 5.36 (d, 2H, J=46.8Hz), 3.10-3.00 (s, 1H).

### Example 2:

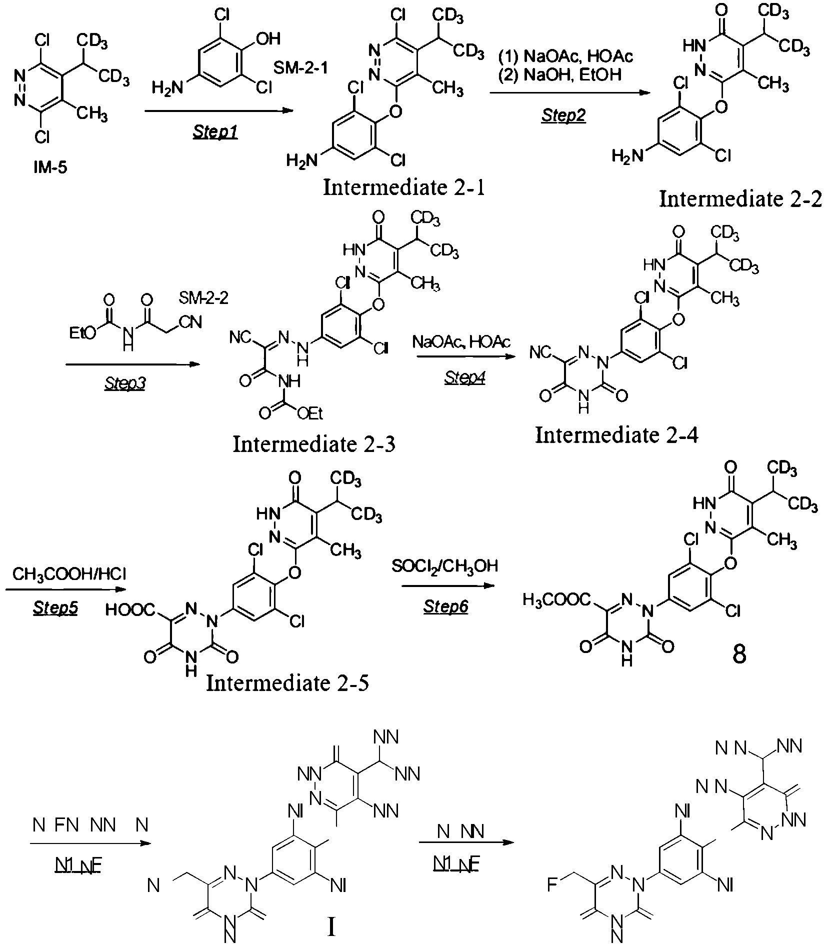

### First step:

IM-5 (7.3 g), SM-2-1 (6.2 g), cuprous iodide (3.3 g), potassium carbonate (12.1 g), and 75 mL of dimethyl sulfoxide were sequentially added to a reaction flask, and heated to 90°C after nitrogen protection, and a reaction was carried out for 5 h. The reaction solution was cooled and added into water, filtration was performed through diatomaceous earth, a filter cake was rinsed with methanol, a filtrate and a solution obtained after rinsing were mixed and extracted with ethyl acetate (500 mL×2), organic phases were mixed, washed with a saturated aqueous sodium chloride solution, dried over anhydrous sodium sulfate, concentrated under reduced pressure to dryness, and purified by Flash chromatography to give 4.8 g of an intermediate 2-1 as an off-white solid. m/z: 352.05 (M+1), 350.05 (M-1).

### Second step:

The intermediate 2-1 (4.8 g), sodium acetate (5.65 g) and 40 mL of acetic acid were sequentially added to a reaction flask and heated to 130°C, and a reaction was carried out for 7.0 h. After the reaction solution was cooled, the reaction solution was concentrated under reduced pressure to dryness, 20 mL of ethyl acetate was added to dissolve the concentrated material, and then the dissolved material was concentrated under reduced pressure again to dryness, and the obtained concentrate was directly used for a reaction of a next step without further purification. m/z: 376.10 (M+1), 374.15 (M-1).

A crude obtained in the above step and 50 mL of ethanol were added to a reaction flask, a solution of sodium hydroxide (8.3 g) in water (20 mL) was then added, heating was performed to 110°C, and a reaction was carried out for 4 h. After the reaction solution was cooled, the reaction solution was concentrated under reduced pressure to dryness, and 50 mL of water and 50 mL of ethyl acetate were added to the obtained concentrate, and stirring and liquid separation were performed. An aqueous layer was further extracted with ethyl acetate (50 mL×2), and ethyl acetate layers were mixed, sequentially washed with 100 mL of a saturated aqueous sodium chloride solution, dried over anhydrous sodium sulfate, and concentrated under reduced pressure to dryness. The obtained concentrate was slurried with a mixed solution of ethyl acetate and petroleum ether (1:2), filtered, and oven-dried to give 3.5 g of a yellow-brown solid. m/z: 334.05 (M+1), 332.10 (M-1).

### Third step:

An intermediate 2-2 (3.5 g) was dissolved in 150 mL of water, the obtained solution was cooled to 0-10°C after adding 177 mL of concentrated hydrochloric acid, a solution of sodium nitrite (2.2 g) in water (15 mL) was added while maintaining T=0-10°C, and stirring was performed for 1 h under the ice-water bath condition after completion of the addition. The obtained solution was recorded as a solution A. SM-2-2 (1.98 g) was added to 75 mL of water, followed by addition of 177 mL of pyridine, and the obtained solution was recorded as a solution B. The solution A was slowly added to the solution B at a temperature controlled to be T=0-10°C, and a reaction was carried out for 1.5 h while maintaining T=0-10°C after the dropwise addition was complete. The reaction solution was filtered, and a filter cake was washed with water and n-hexane and oven-dried to give 4.7 g of an orange-red solid. m/z: 501.20 (M+1), 499.15 (M-1).

### Fourth step:

An intermediate 2-3 (4.7 g), sodium acetate (7.85 g) and acetic acid 145 mL were added to a reaction flask and heated to 130°C, and a reaction was carried out for 3 h. The reaction solution was cooled, and concentrated under reduced pressure to dryness, 100 mL of water was added, the obtained solution was extracted with ethyl acetate (75 mL×3), and ethyl acetate layers were mixed, successively washed with 200 mL of a saturated aqueous sodium chloride solution, dried over anhydrous sodium sulfate, and concentrated under reduced pressure to dryness. The obtained concentrate was slurried with a mixed solution of ethyl acetate and n-hexane (1:2), filtered, and oven-dried to give 3.5 g of a light brick-red solid. m/z: 455.10 (M+1), 453.10 (M-1).

### Fifth step:

An intermediate 2-4 (3.5 g) and 53 mL of acetic acid were added to a reaction flask, concentrated hydrochloric acid (24 mL) was added, heating was performed to 120°C, and a reaction was carried out for 2.5 h. After the reaction solution was cooled to ambient temperature, the reaction solution was poured into 110 mL of water, stirring was performed for 0.5 h and filtration was performed. A filter cake was washed with water and oven-dried to give 3.2 g of a light brick-red solid. m/z: 474.05 (M+1), 472.05 (M-1).

### Sixth step:

An intermediate 2-5 (3.2 g) and 60 mL of methanol were added to a reaction flask, thionyl chloride (820 mg) was added slowly with stirring, heating was performed to 100°C, and a reaction was carried out for 4 h. After the reaction solution was cooled to room temperature, the reaction solution was concentrated under reduced pressure to dryness, and the obtained concentrate was slurried with a mixed solution of ethyl acetate and petroleum ether (1:2), filtered, and oven-dried to give 3.0 g of a compound 8 as a light brick-red solid. m/z: 488.10 (M+1), 486.10 (M-1).

### Seventh step:

The compound 8 (3.0 g) was dissolved in a mixed solution of 150 mL of tetrahydrofuran and 15 mL of methanol, sodium borohydride (1.87 g) was added slowly with stirring, and a reaction was carried out at room temperature for 0.5 h. The reaction was quenched by adding 90 mL of water to the reaction solution, the reaction solution was adjusted to pH=6 with hydrochloric acid (1 M), and concentrated to about half the volume remaining, the concentrated reaction solution was extracted with ethyl acetate (90 mL×3), and ethyl acetate layers were mixed, sequentially washed with 200 mL of a saturated aqueous sodium chloride solution, dried over anhydrous sodium sulfate, and concentrated under reduced pressure to dryness. The obtained concentrate was slurried with a mixed solution of ethyl acetate and n-hexane (1:1), filtered, and oven-dried to give 2.6 g of a compound 12 as an off-white solid. M/z: 460.10 (M+1), 458.05 (M-1).

### Eighth step:

The compound 12 (2.4 g) and 200 mL of dichloromethane were added to a reaction flask, diethylaminosulfur trifluoride (DAST, 2.5 g) was slowly added at T=0-5°C, and after the dropwise addition was completed, a reaction was carried out at room temperature for 0.5 h. The reaction was quenched by adding 200 mL of ice-water to the reaction solution, stirring and liquid separation were performed, and an aqueous phase was extracted with dichloromethane (100 mL×2), dichloromethane layers were mixed, sequentially washed with 300 mL of a saturated aqueous sodium chloride solution, dried over anhydrous sodium sulfate, and concentrated under reduced pressure to dryness. The obtained concentrate was purified by a Flash chromatography column to give 395 mg of a white solid. m/z: 462.10 (M+1), 460.10 (M-1); ¹H NMR (600 MHz, DMSO) δ 12.00 (s, 1H), 7.81 (s, 2H), 5.30 (d, *J =* 46.8 Hz, 2H), 3.25 (s, 1H), 2.32 (d, *J =* 10.5 Hz, 3H); ¹³C NMR (150 MHz, DMSO) δ 159.74, 156.59, 150.93, 148.41, 147.82, 145.36, 141.17, 141.07, 138.45, 131.15, 128.42, 126.91, 79.55, 78.44, 27.99, 18.40, 12.64.

### Example 3:

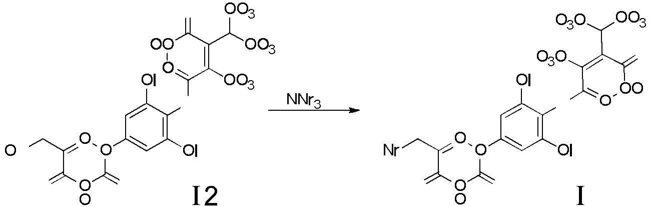

The compound 12 (60 mg) and tetrahydrofuran were added to a reaction flask, stirring was performed at ambient temperature until a clear solution was obtained, phosphorus tribromide (71 mg) was added, heating was performed to 50°C, and a reaction was carried out for 0.5 h. After the reaction solution was cooled, the reaction solution was subjected to column chromatography to prepare 48 mg of a white solid. m/z: 524.05 (M+1), 522.05 (M-1).

### Example 4:

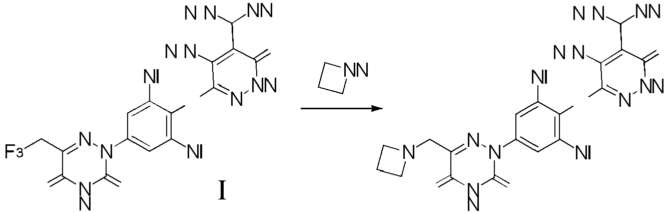

A compound 10 (290 mg), azetidine (40 mg), and potassium carbonate (116 mg) were weighed, toluene (15 mL) and 1,4-dioxane (5 mL) were added, heating was started to 50°C, and a reaction was carried out for 2 h. The reaction solution was filtered, and a filtrate was concentrated to dryness and purified by column chromatography to give 25mg of a white solid. m/z: 500.10 (M+H), 499.05 (M-H); ¹H-NMR (600 MHz, DMSO-d₆) δ: 12.20 (s, 1H), 7.80 (s, 2H), 3.57 (s, 2H), 3.37 (t, 4H, J =21.0, 10.2 Hz), 3.09-3.00 (s, 1H), 2.34 (s, 3H), 2.06-1.99 (m, 2H).

### Example 5:

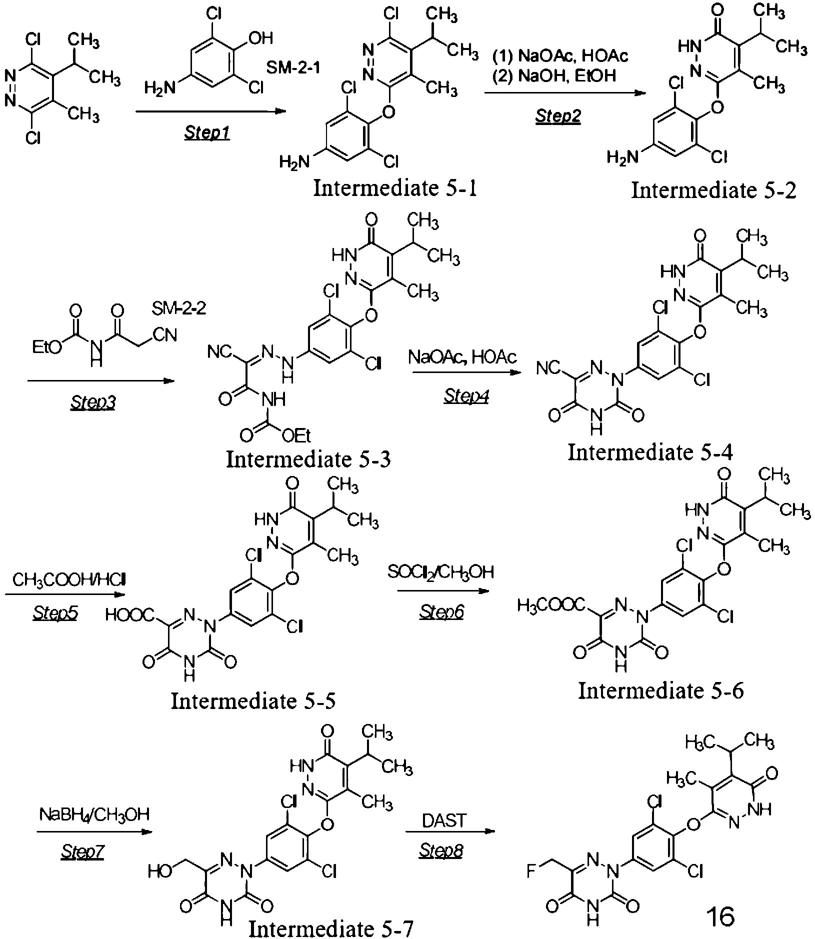

### First step:

3,6-Dichloro-4-isopropyl-5-methylpyridazine (1.0 g), SM-2-1 (873 mg), cuprous iodide (466 mg), potassium carbonate (1.69 g), and 10 mL of dimethyl sulfoxide were sequentially added to a reaction flask, and heated to 90°C after nitrogen protection, and a reaction was carried out for 16 h. The reaction solution was cooled, added to water, and extracted with EtOAc (50 m1×2), and organic phases were mixed, washed with a saturated salt solution, dried over anhydrous sodium sulfate, concentrated, and purified by column chromatography to give 400 mg of a yellow oil. m/z: 348.05 (M+1).

### Second step:

An intermediate 5-1 (800 mg), sodium acetate (951 mg) and 8 mL of acetic acid were sequentially added to a reaction flask, and heated to 130°C, and a reaction was carried out for 16 h. The reaction solution was cooled, concentrated to dryness, and directly used in a reaction of a next step without purification. m/z: 370.05 (M+1), 368.05 (M-1).

A product obtained in the previous step and 20 mL of ethanol were added to a reaction flask, followed by addition of a solution of sodium hydroxide (9.28 g) in water (10 mL), heating was performed to 110°C, and a reaction was carried out for 3 h. The reaction solution was cooled, and concentrated to semi-dryness, water was added to the semi-dried material for slurrying, and filtration was performed. A filter cake was washed with ethyl acetate and concentrated to dryness to give 600 mg of a yellow-brown solid. m/z: 328.00 (M+1), 326.05 (M-1).

### Third step:

An intermediate 5-2 (500 mg) was dissolved in 60 mL of water, 30 ml of hydrochloric acid was added, then the temperature was reduced to 0-10°C, a solution of sodium nitrite (379 mg) in water (3 mL) was added, and stirring was performed for 1 h under the ice-water bath condition after completion of the addition. The obtained solution was recorded as a solution A. SM-2-2 (342 mg) was added to 15 ml of water, followed by addition of 30 mL of pyridine, and the obtained solution was recorded as a solution B. The solution A was slowly added to the solution B at a temperature controlled to be 0-10°C, and after the dropwise addition was complete, stirring was continued to be performed for 1.5 h. The reaction solution was filtered, and a filter cake was washed and oven-dried to give 650 mg of an orange solid. m/z: 495.10 (M+1), 493.10 (M-1).

### Fourth step:

An intermediate 5-3 (650 mg), sodium acetate (1.08 g) and 33 mL of acetic acid were added to a reaction flask, and heated to 130°C, and a reaction was carried out for 3 h. The reaction solution was cooled, and concentrated to dryness, 25 mL of water was added, the reaction solution was extracted with ethyl acetate (25 mL×2), and organic phases were mixed, washed with saturated sodium chloride, dried over anhydrous sodium sulfate, concentrated to dryness, and purified by column chromatography to give 190 mg of a white solid. m/z: 449.00 (M+1), 447.00 (M-1); ¹H-NMR (600 MHz, DMSO-d₆) δ 12.02 (s, 1H), 7.78 (s, 2H), 3.32-3.24 (m, 1H), 2.34 (s, 3H), 1.31 (d, J = 6.6 Hz, 6H).

### Fifth step:

An intermediate 5-4 (350 mg) and 20 mL of acetic acid were added to a reaction flask, concentrated hydrochloric acid (10 mL) was added, heating was performed to 120°C, and a reaction was carried out for 2.5 h. After the reaction solution was cooled to ambient temperature, the reaction solution was poured into 50 mL of water, stirring was performed for 0.5 h, and filtration was performed. A filter cake was washed with water and oven-dried to give 200 mg of a light brick-red solid.

### Sixth step:

An intermediate 5-5 (200 mg) and 20 mL of methanol were added to a reaction flask, thionyl chloride (300 mg) was added slowly with stirring, heating was performed to 100°C, and a reaction was carried out for 4 h. After the reaction solution was cooled to room temperature, the reaction solution was concentrated under reduced pressure to dryness, and the obtained concentrate was slurried with a mixed solution of ethyl acetate and petroleum ether, and the slurried material was filtered, and oven-dried to give 100mg of a light brick-red solid.

### Seventh step:

A compound 5-6 (100 mg) was dissolved in a mixed solution of 15 mL of tetrahydrofuran and 2 mL of methanol, and sodium borohydride (200 mg) was added slowly with stirring, and a reaction was carried out at room temperature for 0.5 h. The reaction was quenched by adding 30 mL of water to the reaction solution, the reaction solution was adjusted to pH=6 with hydrochloric acid (1 M), and concentrated to about half the volume remaining, the concentrated reaction solution was extracted with ethyl acetate (20 mL×3), and ethyl acetate layers were mixed, sequentially washed with 20 mL of a saturated aqueous sodium chloride solution, dried over anhydrous sodium sulfate, and concentrated under reduced pressure to dryness. The obtained concentrate was slurried with a mixed solution of ethyl acetate and n-hexane, and the slurried material was filtered and oven-dried to give 80 mg of a compound as an off-white solid.

### Eighth step:

A compound 5-7 (80 mg) and 20 mL of dichloromethane were added to a reaction flask, diethylaminosulfur trifluoride (DAST, 100 mg) was slowly added at a temperature controlled to be 0-5°C, and after the dropwise addition was completed, a reaction was carried out at room temperature for 0.5 h. The reaction was quenched by adding 20 mL of ice-water to the reaction solution, stirring and liquid separation were performed, and an aqueous phase was extracted with dichloromethane (20 mL×2), dichloromethane layers were mixed, sequentially washed with 30 mL of a saturated aqueous sodium chloride solution, dried over anhydrous sodium sulfate, and concentrated under reduced pressure to dryness, and the obtained concentrate was purified by column chromatography to give 40 mg of a white solid. m/z: 457.36 (M+1); ¹H NMR (600 MHz, DMSO) δ 12.69 (s, 1H), 12.00 (s, 1H), 7.81 (s, 2H), 5.30 (d, *J* = 46.8 Hz, 2H), 3.30 (d, *J* = 7.5 Hz, 1H), 2.33 (s, 3H), 1.30 (d, *J* = 6.7 Hz, 6H).

By using a similar preparation scheme, the following compound can be prepared; 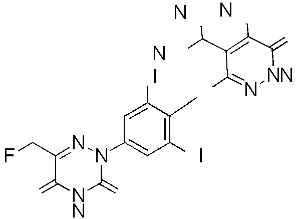 m/z: 457.16 (M+1); ¹H NMR (600 MHz, DMSO) δ 12.68 (s, 1H), 12.14 (s, 1H), 7.81 (s, 2H), 5.30 (d, *J =* 46.8 Hz, 2H), 3.38 (d, *J =* 7.7 Hz, 1H), 2.16 (s, 3H), 1.42 (d, *J =* 6.8 Hz, 6H).

### Example 6:

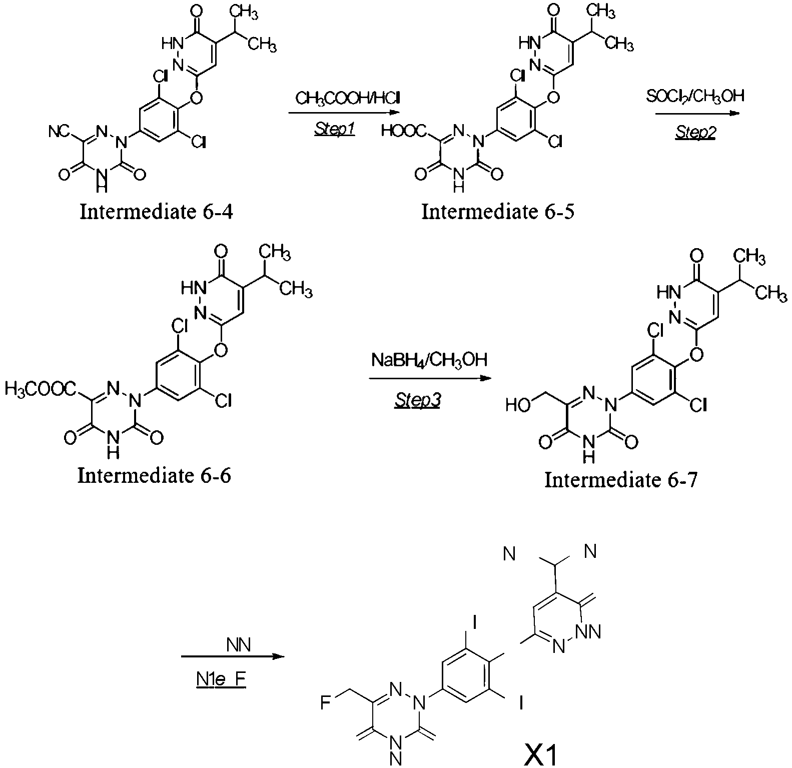

### First step:

An intermediate 6-4 (prepared with reference to Example 2 in WO2021104288A1) (3 g) was dissolved in acetic acid (AcOH, 100 mL), and concentrated hydrochloric acid (20 mL) was added with stirring. After the addition was complete, heating was performed to 120°C, and a reaction was carried out for 6 h. After the reaction was completed, the reaction solution was cooled to room temperature, and H₂O (200 mL) was added dropwise to the reaction solution. After the dropwise addition was complete, stirring was performed at room temperature for 1 h, filtration was performed, and a filter cake was rinsed with H₂O (20 mL×2). A solid was collected and dried at 45°C to give 2.0 g of an earthy yellow solid. m/z: 454.00 (M+H), 452.00 (M-H).

### Second step:

An intermediate 6-5 (2 g) was dissolved in methanol (CH₃OH, 100 mL), and thionyl chloride (SOCl₂, 10 mL) was added dropwise under an ice bath. After the addition was complete, heating was performed to 100°C, and a reaction was carried out for 5 h. After completion of the reaction, the reaction solution was concentrated under reduced pressure. A residue was slurried with petroleum ether and ethyl acetate in a ratio of 1:1 for 1 h, and filtration was performed. A filter cake was rinsed with petroleum ether. A solid was collected and dried at 45°C to give 2.3 g of a yellow solid. m/z: 468.00 (M+H), 466.00 (M-H); ¹H-NMR (400 MHz, DMSO-d6) δ: 12.77 (s, 1H), 12.21 (s, 1H), 7.81 (s, 2H), 7.45 (d, 1H, *J* =1.2 Hz), 3.84 (s, 3H), 3.10-3.00 (m, 1H), 1.21 (s, 3H), 1.19 (s, 3H).

### Third step:

An intermediate 6-6 was dissolved in tetrahydrofuran (THF, 40 mL), sodium borohydride (NaBH₄, 0.82 g) was added, methanol (4 mL) was slowly added dropwise after the addition was complete to generate a large amount of bubbles, and a reaction was carried out for 1 h with stirring. After completion of the reaction, the reaction was quenched with ice water, and the reaction solution was adjusted to a pH of about 6 with 1 M HCl, and extracted with ethyl acetate (20 mL×2), organic phases were mixed, washed with saturated NaCl (20 mL), dried over anhydrous sodium sulfate, filtered, and concentrated, and a residue was allowed to pass through a column (dichloromethane:methanol=15:1-10:1) to give 1.6 g of a light yellow solid. m/z: 440.00 (M+H), 438.05 (M-H); ¹H-NMR (400 MHz, DMSO-d6) δ: 12.44 (s, 1H), 12.21 (s, 1H), 7.86 (s, 2H), 7.44 (d, 1H, *J* =0.8 Hz), 5.31-5.27 (t, 1H, *J* =12.4&6.4 Hz),4.41 (d, 2H, *J* =5.6 Hz), 3.10-3.00 (m, 1H), 1.21 (s, 3H), 1.19 (s, 3H).

### Fourth step:

An intermediate 6-7 (100 mg) was dissolved in dichloromethane (20 mL), diethylaminosulfur trifluoride (DAST, 8 drops) was added dropwise under an ice bath, and stirring was performed for 30 min under the ice bath. After completion of a reaction, the reaction solution was slowly added dropwise into ice water, liquid separation was performed, and an organic phase was temporarily stored. An aqueous phase was extracted with dichloromethane (15 mL). Organic phases were mixed, washed with saturated NaCl (20 mL), dried over anhydrous sodium sulfate, filtered, and concentrated, and a residue was slurried with EA to give 20 mg of a compound X1 as a white solid. m/z: 442.00 (M+H), 440.05 (M-H); ¹H-NMR (400 MHz, DMSO-*d6*) δ: 12.67 (s, 1H), 12.22 (s, 1H), 7.82 (s, 2H), 7.44 (d, 1H, *J*=0.8 Hz), 5.36 (d, 2H, *J*=46.8 Hz), 3.10-3.00 (m, 1H), 1.21 (s, 3H), 1.19 (s, 3H).

### Biological test

### 1. Experiment of binding of compounds to THRβ and THRα

**Test principle:** The effect of test compounds on THRβ and THRα was tested by using a method of HTRF. An MAb Anti GST-Eu cryptate antibody binds to GST-tagged THRα-LBD (or THRβ-LBD), while Streptavidin-XL665 binds to a biotin-labeled SRC3-2 coactivator peptide. The binding of an agonist to THRα-LBD (THRβ-LBD) results in a change in the conformation of THRα-LBD (THRβ-LBD), thereby increasing its ability to recruit the SRC3-2 coactivator peptide. At the same time, the resulting decrease in the distance between XL665-labeled SRC3-2 and an Eu-anti-GST antibody increases a THRFRET signal. The agonistic capacity at this target was assessed by the effect of compounds on the activity of THRα-LBD (THRβ-LBD) at different concentrations.

### Test method: THRβ test procedures:

(1) a 1×reaction solution was prepared.
(2) Preparation of compounds: the compound test starting concentration was 20 µM, 3-fold dilution was performed, 10 concentrations were set, and duplicate testing was set for each concentration. 100-fold dilution was performed in a 384-well Source plate to a corresponding solution with a final concentration, and then 200 nL of the solution was transferred to a 384-well reaction plate with Echo550 to be tested. 200 nL of 100% DMSO and a test article were respectively transferred to Min and Max wells.
(3) A 2×protein solution was prepared from the 1×reaction solution.
(4) A 4×SRC3-2 solution was prepared from the 1×reaction solution.
(5) A mixed solution of 4×GST-Eu cryptate and XL665 was prepared from the 1×reaction solution.
(6) 10 µl of the 2×protein solution was added to each well of the reaction plate, centrifugation was performed at 1000 rpm for 1 min, and incubation was performed at room temperature for 30 min.
(7) 5 µl of the 4×SRC3-2 solution was added to each well of the reaction plate, and centrifugation was performed at 1000 rpm for 1 min.
(8) 5 µL of the mixed solution of 4×GST-Eu cryptate and XL665 was added to each well of the reaction plate, centrifugation was performed at 1000 rpm for 1 min, and incubation was performed at 25°C.
(9) A fluorescence signal was read by using EnVision.

THRα test procedures were the same as above.

### Test results:

### Activity of compounds of the present invention on THRβ and THRα

| **Compound No. in Examples** | **THR**β **(EC₅₀, nM)** | **THR**α **(EC₅₀, nM)** |
|---|---|---|
| 1 | 20 | 326 |
| 6 | 6.5 | >5000 |
| 16 | 11 | >5000 |
| 17 | 46 | >5000 |
| X1 | 22 | 95 |
| Resmetirom | 166 | 2985 |

The above data show that compounds 1, 6, 16, and 17 of the present invention have a better agonistic effect on THRβ and its agonistic activity on THRβ is significantly better than that of Resmetirom; on the other hand, compounds 6, 16, and 17 have weak agonistic activity on THRα, with little agonistic activity, indicating that compounds 6, 16, and 17 have selective agonistic effects on THRβ and excellent safety.

### 2. Study on comparison of compound metabolites by a human liver microsome incubation method

The purpose of this experiment was to evaluate the comparison of the content of major metabolites of human liver microsome incubated compounds.

### Human liver microsome incubation:

| | |
|---|---|
| Compound concentration: | 10 µM |
| Microsomal protein concentration: | 1.0 mg/mL |
| Microsomal strains: | Human |
| *β-NADPH concentration:* | 1.0 mM |
| Phosphate buffer: | 50 mM, pH 7.4 |
| Incubation temperature: | 37°C |
| Incubation time: | 0 min and 60 min |
| Positive control: | 7-Ethoxycoumarin (7-EC; 0 min and 60 min) |

Sample treatment: a sample was precipitated with acetonitrile (ACN) containing 0.1% formic acid in a ratio of 1:2, and further centrifuged. A supernatant was dried with a nitrogen flow. A residue was reconstituted in 300 L of 10% ACN and 90% H₂O containing 0.1% formic acid. 10 L of the reconstituted material was injected into an LC-MS/MS system for analysis.

### Sample chromatography

| | |
|---|---|
| Column model: | ACQUITY UPLC HSS T3, 1.8 µm, 2.1×100 mm |
| Column temperature: | 25°C |
| Flow rate: | 0.3 mL/min |
| Injection volume: | 10 µL |
| Run time: | 22 min |
| Mobile phase: | A=Water containing 0.1% formic acid |
| | B=Acetonitrile containing 0.1% formic acid |

### Gradient elution table

| | Time (min) | A (%) | B (%) |
|---|---|---|---|
| | 1.0 | 95 | 5 |
| | 6.0 | 60 | 40 |
| HPLC gradient: | 14.0 | 20 | 80 |
| | 16.0 | 10 | 90 |
| | 18.0 | 5 | 95 |
| | 20.1 | 95 | 5 |
| | 22.0 | 95 | 5 |

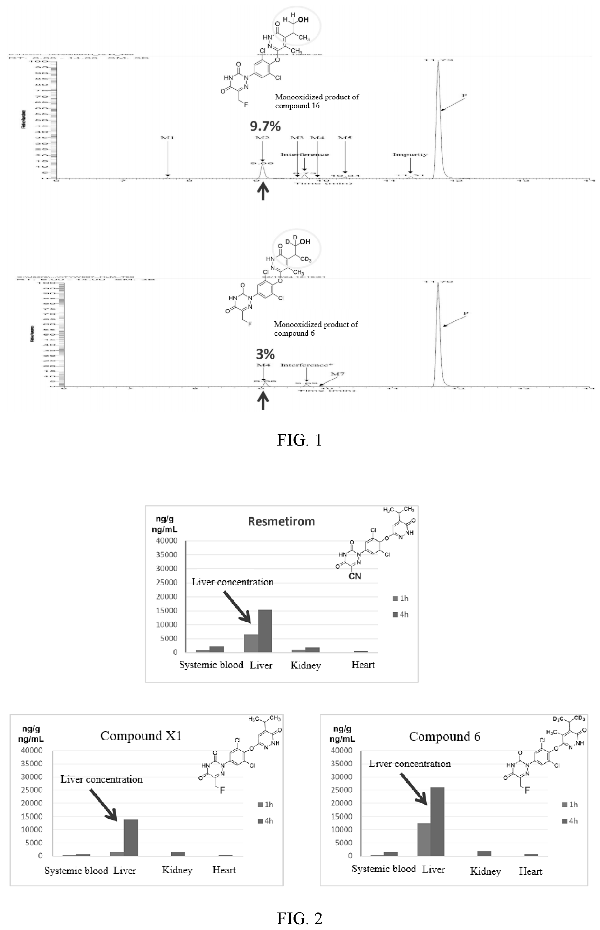

Sample test results: Specific test spectra are shown in FIG. 1 and data is as follows.

### Study on the percent content of major oxidative metabolite of compounds by human liver microsome incubation (%)

| **Compound** | **Monooxidized metabolite percent content (%)** |
|---|---|
| Compound 16 | 9.7% |
| Compound 6 | 3.0% |

Data from the above study show that at the same study dose and in the same human liver microsome incubation system, the content of a major metabolite of the compound 16 (a monooxidized product: 9.7%) is significantly higher than that of a major metabolite of the compound 6 (a monooxidized product: 3%), indicating that the substitution of hydrogen atoms in methyl in the compound 6 by deuterium atoms significantly increases the metabolic stability.

### 3. In vivo pharmacokinetic study of the elimination phase half-life (T_{1/2}) characteristics of compounds in Sprague Dawley rats

The purpose of this experiment was to evaluate the elimination phase half-life characteristics of test compounds after single IV bolus administration.
Route of administration: Group 1: Resmetirom IV bolus; Group 2: Compound 6 IV bolus;
Administration dosage: Group 1: 5 mg/kg; Group 2: 5 mg/kg;
Dosing frequency: single dose;
Administration method: animals were administered by tail vein injection by accurately sucking a test article at a desired concentration by using a suitable sterile disposable syringe and an intravenous infusion needle, wherein administration was completed at approximately 30 seconds.
Sample collection: Approximately 0.3 mL of whole blood was collected from jugular veins of animals in each group at each time point. Blood samples were collected from animals (IV group) before administration and at time points of 0.083 h, 0.25 h, 0.5 h, 1 h, 2 h, 4 h, 8 h, and 24 h after the end of administration.
Sample treatment: prior to blood collection, a centrifuge tube with 10 µl of EDTA-K₂ and 1.2 mL of 50% acetonitrile-DMSO (a volume ratio of whole blood to 50% acetonitrile-DMSO is approximately 1:4) was placed in an ice box filled with crushed ice. The collected blood was added to the centrifuge tube, vortexed for at least 5 min, and then temporarily stored and transported in the ice box filled with crushed ice; and the blood was centrifuged at 12,000 g for 10 min at 4°C, wherein centrifugation was completed within 1 h after blood collection. A whole blood supernatant after centrifugation was transferred to a newly labeled tube, and transferred to -60°C or below for storage for analytical testing.

### In vivo pharmacokinetic study of the elimination phase half-life (T_{1/2}) of compounds in rats

| **Compound** | **Administration mode** | **Administration dosage (mg/kg)** | **Elimination phase half-life (T_{1/2})** |
|---|---|---|---|
| Resmetirom | I.V | 5 | 2.98 |
| Compound 6 | I.V | 5 | 7.80 |

The above study data indicate that the elimination phase half-life of the compound 6 (T_{1/2}=7.80 h) is significantly longer than the elimination phase half-life of Resmetirom (T_{1/2}=2.98 h) in the same study animal system at the same study dose.

### 4. Study on the distribution of compounds in major organs of Sprague Dawley rats

The purpose of this experiment was to evaluate the distribution characteristics of test compounds in major organs after single bolus gavage administration.
Route of administration: Group 1: Resmetirom gavage administration; Group 2: Compound X1 gavage administration; Group 3: Compound 6 gavage administration;
Administration dosage: Group 1: 5 mg/kg; Group 2: 5 mg/kg; Group 3: 5 mg/kg; Dosing frequency: single dose

### Blood collection and organs:

0.25-0.3 ml of Systemic blood (jugular vein) was taken at each blood collection point to be added into an EDTA-K2 anticoagulant tube, uniform mixing was performed by gentle inversion, and the obtained mixture was stored on crushed ice for transport. Collection plan: PO blood collection point: 1 h and 4 h after administration. Animals were euthanized, tissues and organs were taken, and livers (0.3-0.5 g of liver was taken at a fixed position of a large lobe of liver), kidneys, and hearts were weighed, placed in 5 ml centrifuge tubes, quick-frozen on dry ice, and cryopreserved at -20°C.

### Sample pre-treatment:

A solvent (methanol:acetonitrile:water=25:25:50) was added for grinding and homogenization, and this process was performed rapidly by using a tissue grinder.

A solvent was added to the liver to 3 ml, one large steel ball and one small steel ball were added, and grinding was performed at 50 Hz for 60 s. Vortexing was performed for uniform mixing, and centrifugation was performed at 8000 rpm for 5 min. 0.3 ml of a supernatant was taken, a solvent was added until 3 ml, vortexing was performed for uniform mixing,, and centrifugation was performed at 8000 rpm for 5 min. 0.1 ml of a supernatant was taken, 0.2 ml of acetonitrile was added, vortexing was performed for 30 s, and centrifugation was performed at 10000 rpm for 5 min. A supernatant was taken and sent for testing to analyze the drug content.

A solvent was added to the kidney to 3 ml in each of two centrifuge tubes, one large steel ball and one small steel ball were added, grinding was performed at 50 Hz for 60 s, the grinding effect was observed, and grinding can be performed for multiple times. After grinding, vortexing was performed for uniform mixing, and centrifugation was performed at 8000 rpm for 5 min. 0.3 ml of a supernatant was taken from each of the two centrifuge tubes, a solvent was added until 3 ml, vortexing was performed for uniform mixing, and centrifugation was performed at 8000 rpm for 5 min. 0.1 ml of a supernatant was taken, 0.2 ml of acetonitrile was added, vortexing was performed for 30 s, and centrifugation was performed at 10000 rpm for 5 min. A supernatant was taken and sent for testing to analyze the drug content.

A solvent was added to the heart to 3 ml, one large steel ball and one small steel ball were added, grinding was performed at 50 Hz for 30 s, and the ground heart was taken out and cut into pieces with scissors. Grinding was performed at 50 Hz for 60 s, and the grinding effect was observed, and grinding can be performed for multiple times. After grinding, vortexing was performed for uniform mixing, and centrifugation was performed at 8000 rpm for 5 min. 0.1 ml of supernatant was taken, 0.2 ml of acetonitrile was added, vortexing was performed for 30 s, and centrifugation was performed at 10000 rpm for 5 min. A supernatant was taken and sent for testing to analyze the drug content.

0.1 ml of plasma was taken, 0.2 ml of acetonitrile was added, vortexing was performed for 30 s, and centrifugation was performed at 10000 rpm for 5 min. A supernatant was taken and sent for testing to analyze the drug content.

### Detection method:

Chromatographic column: C18 Ultimate UHPLC XB-C18 (2.1×50 mm) 377# with a protective column of the same model
Mobile phase: A: a 0.1% formic acid solution; B: Acetonitrile
Gradient elution

| Time/min | A% | B% |
|---|---|---|
| 0 | 60 | 40 |
| 3 | 60 | 40 |
| 10 | 45 | 55 |
| 12 | 10 | 90 |
| 13.5 | 10 | 90 |
| 13.6 | 60 | 40 |
| 15 | 60 | 40 |

Flow rate: 0.2 mL/min
Column temperature: 40°C
Injection volume: 10 µL

### Results and analysis:

According to the test data, the summary chart is shown in FIG 2.

### Test conclusion:

Data from the above study indicate that the compound 6 of the present invention has a significant liver distribution advantage over Resmetirom and the compound X1.

### 5. Efficacy test experiment of compounds in NASH model of B-DIO mice

### Test objective:

An objective of this experiment was to evaluate the pharmacodynamics of compounds of the present invention by using an NASH model of B-DIO mice.

### Test method:

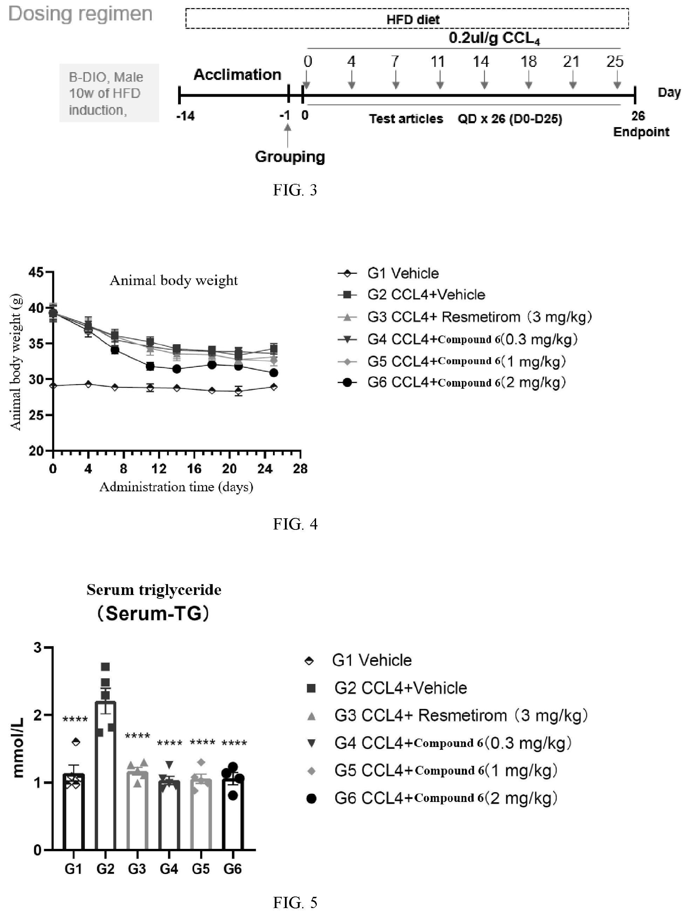

Five C57BL/6 mice on normal diet were assigned to an experimental group G1, and 25 B-DIO mice were randomly assigned to five experimental groups according to the body weight, with 5 mice in each group. A dosing regimen is shown in FIG. 3. The experimental groups were shown in the following table:

| Grou p | Modeling mode | Test article | Numb er of animal s | Dose (mg/k g) | Route of Administrati on | Dosing frequenc y | Times of administrati on |
|---|---|---|---|---|---|---|---|
| G1 | NA | Vehicle | 5 | - | I.G. | Q.D. | 26 |
| G2 | HFD+CC L₄ | Vehicle | 5 | - | I.G. | Q.D. | 26 |
| G3 | HFD+CC L₄ | Resmetiro m | 5 | 3 | I.G. | Q.D. | 26 |
| G4 | HFD+CC L₄ | Compoun d 6 | 5 | 0.3 | I.G. | Q.D. | 26 |
| G5 | HFD+CC L₄ | Compoun d 6 | 5 | 1 | I.G. | Q.D. | 26 |
| G6 | HFD+CC L₄ | Compoun d 6 | 5 | 2 | I.G. | Q.D. | 26 |

The day of grouping was Day-1, intraperitoneal injection was started on Day 0 in the groups G2-G6 for CCL₄ modeling, the concentration of a modeling agent is 0.2 µl/g, the modeling frequency was twice a week, and administration was started on Day 0.

The body weight was measured twice a week after grouping. At the end of the experiment (Day 26), the mice were fasted for 4 h, and non-anticoagulated blood of the mice in the groups G1-G6 was collected and centrifuged, and a serum was taken for blood biochemical testing. Livers in the groups G1-G6 were collected and weighed, 20 mg of the liver was weighed for determination of TC and TG, and the remaining liver was placed in formalin for fixation for pathological observation.

### Results and analysis:

There were no abnormal deaths or significant clinical symptoms of the animals during the study. On Day 26 of grouping, the average body weight in the model group G2 was significantly higher than that of the vehicle control group G1. The body weight did not change significantly in the remaining dosing groups compared with the model group G2, as shown in FIG. 4.

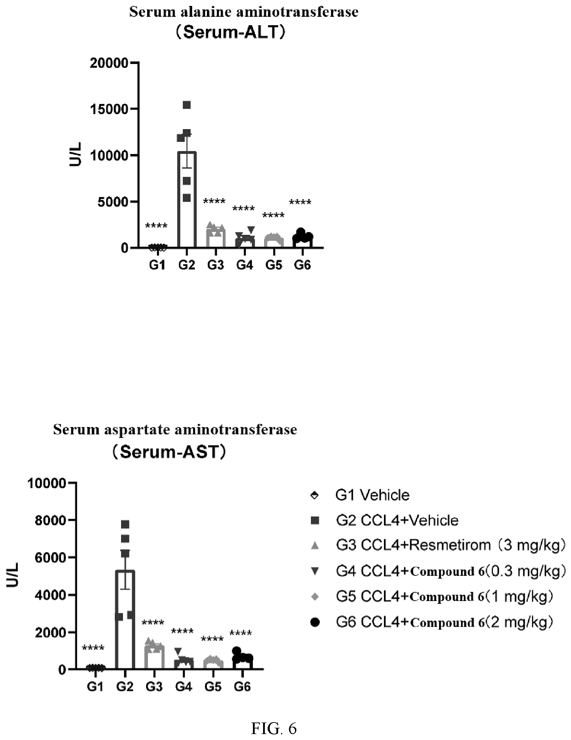

On Day 26, the values of ALT, AST, and TG in the model group G2 were significantly increased compared with the vehicle control group G1, and the above indicators of the mice in the administration groups G3-G6 were significantly reduced compared with the model group G2, wherein the value of ALT was decreased by more than 80%, the value of AST was decreased by more than 75%, and the value of TG was decreased by more than 45%, and the specific values are shown in the following table, and are also shown in FIGS. 5 and 6.

### Effect of test articles on blood lipid and liver injury indicators in NASH mice induced by HFD combined with carbon tetrachloride on Day 26

| Grou p | Modeling mode | Test article | Dose (mg/k g) | ALT (U/L) | AST (U/L) | TG (mM) |
|---|---|---|---|---|---|---|
| G1 | NA | Vehicle | - | 31.90±0.85 | 88.46±4.17**** | 1.14±0.12**** |
| G2 | HFD+CC L₄ | Vehicle | - | 10460.20±1818 .89 | 5341.00±1040 .65 | 2.21±0.19 |
| G3 | HFD+CC L₄ | Resmetir om | 3 | 2028.20±176.3 4**** | 1296.30±91.8 1**** | 1.17±0.06 **** |
| G4 | HFD+CC L₄ | Compoun d 6 | 0.3 | 1050.00±260.8 4**** | 534.52±114.3 3**** | 1.03±0.06 **** |
| G5 | HFD+CC L₄ | Compoun d 6 | 1 | 1080.58±88.36 **** | 501.72±42.35* *** | 1.06±0.07 **** |
| G6 | HFD+CC L₄ | Compoun d 6 | 2 | 1230.88±155.6 3**** | 709.98±100.5 3**** | 1.06±0.09 **** |

| | | | | | | |
|---|---|---|---|---|---|---|
| Note: a: mean±standard error; b: Statistical analysis (One-way ANOVA) of ALT, AST, and TG in the vehicle control group G1, the administration groups G3-G6 and the model group G2, *p<0.05, **p<0.01, ***p<0.001, ****p<0.0001. | | | | | | |

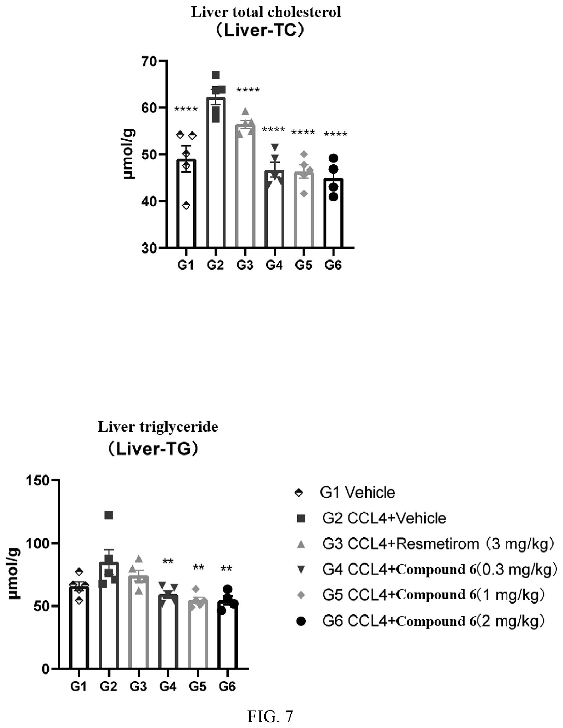

On Day 26, the value of TC was significantly increased in the model group G2 compared with the vehicle control group G1, and the above indicators were significantly decreased in the administration groups G3-G6 by 9.3%, 24.9%, 25.5%, and 27.7%, respectively compared with the model group G2. The value of TG was not significantly changed in the model group G2 compared with the vehicle control group G1, and the above indicators were significantly decreased in the administration groups G4-G6 by 30.4%, 35.9% and 35.9%, respectively compared with the model group G2. The specific values are shown in the following table, and are also shown in FIG. 7.

### Effect of the test articles on TC and TG in the liver of the NASH mice induced by HFD combined with carbon tetrachloride on Day 26

| Group | Modeling mode | Test article | Dose (mg/kg) | TC (µmol/g) | TG (µmol/g)^{ab} |
|---|---|---|---|---|---|
| G1 | NA | Vehicle | - | 49.05±2.79**** | 65.51±3.68 |
| G2 | HFD+CCL₄ | Vehicle | - | 62.29±1.66 | 84.88±9.92 |
| G3 | HFD+CCL₄ | Resmetirom | 3 | 56.44±0.85**** | 74.26±4.34 |
| G4 | HFD+CCL₄ | Compound 6 | 0.3 | 46.76±1.53**** | 59.02±2.79** |
| G5 | HFD+CCL₄ | Compound 6 | 1 | 46.35±1.39**** | 54.34±2.45** |
| G6 | HFD+CCL₄ | Compound 6 | 2 | 44.99±1.87**** | 54.34±3.54** |

| | | | | | |
|---|---|---|---|---|---|
| Note: a: mean±standard error; b: Statistical analysis (One-way ANOVA) of the content of TC and TG in liver in the vehicle control group G1, the administration groups G3-G6 and the model group G2, *p<0.05, **p<0.01, ***p<0.001, ****p<0.0001. | | | | | |

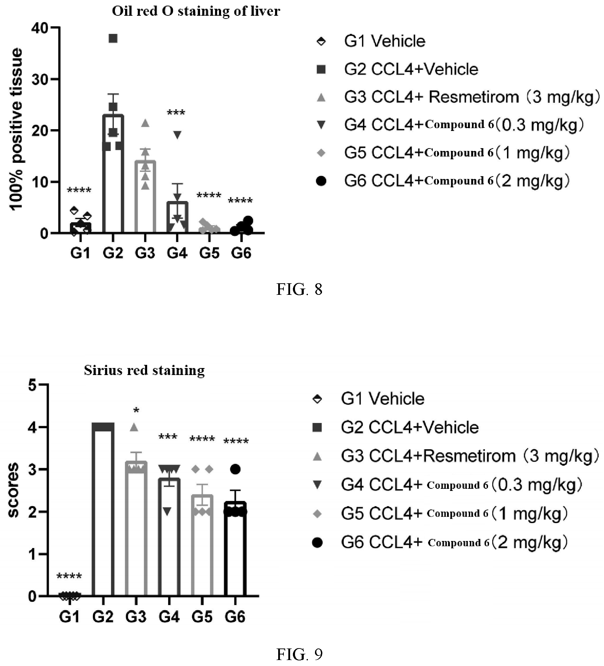

On Day 26, histopathological oil red O detection was performed on the livers of the animals in the groups G1-G6. There was no obvious staining in the vehicle control group G1, and the area proportion of lipid droplets in the liver of the mice in the model group G2 was significantly increased. Compared with the model group G2, the area proportion of lipid droplets of the test article, namely the compound 6 (0.3 mg/kg) (G4), the test article, namely the compound 6 (1 mg/kg) (G5) and the test article, namely the compound 6 (2 mg/kg) (G6) in the livers of the animals was decreased significantly, and the area proportion of lipid droplets of the test article Resmetirom (3 mg/kg) (G3) in the livers of the animals was decreased but no significant difference was seen. It was suggested that the test article, namely the compound 6 had a significant improvement effect on liver steatosis at doses of 0.3 mg/kg, 1 mg/kg and 2 mg/kg, and the test article Resmetirom had a slight improvement effect on liver steatosis at a dose of 3 mg/kg under the experimental conditions, as shown in FIG. 8.

On Day 26, histopathological Sirius red detection was performed on the livers of the animals in the groups G1-G6. The vehicle control group G1 had normal liver tissue with no fibroplasia, while the model group G2 had fibroplasia in a portal area of the liver generally visible under a microscope, accompanied by obvious fiber bridging. The test articles Resmetirom (3 mg/kg) (G3), the compound 6 (0.3 mg/kg) (G4), the compound 6 (1 mg/kg) (G5) and the compound 6 (2 mg/kg) (G6) all had certain improvement effects on liver fibrosis compared with the model group G2, as shown in FIG. 9.

### Test conclusion:

In this experiment, the NASH model of C57 mice was successfully constructed by high-fat diet feeding in combination with CCL₄ injection, and efficacy test experiments of both test articles were successfully completed. Both the test article Resmetirom (G3) and the compound 6 (G4-G6) can greatly improve the increase in transaminase and abnormal blood lipids and liver lipids in mice due to modeling, which is manifested by the ability to significantly reduce ALT, AST, and TG in blood and TC and TG in liver. Additionally, the test articles Resmetirom (G3) and the compound 6 (G5-G6) have a significant reducing effect on hepatic steatosis; resmetirom (G3) and the compound 6 (G4-G6) have a significant improvement effect on liver fibrosis; and the effect of the compound 6 (G4-G6) in reducing the area proportion of liver lipid droplets is significant. The test article, namely the compound 6 (G4-G6) shows better effects of reducing liver fat accumulation and inhibiting liver fibroplasia than Resmetirom (G3) under the same experimental conditions.

## Claims

1. A compound of a formula (X), or a stereoisomer, tautomer, or pharmaceutically acceptable salt thereof, 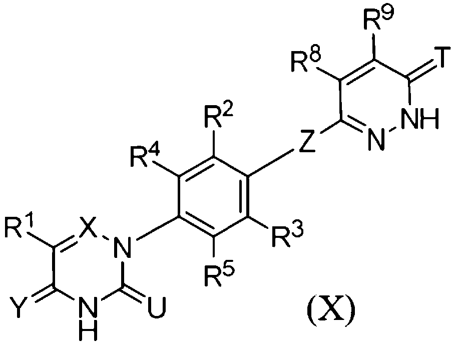
wherein X is N or C;
Y, U and T are each independently N, O or S;
-Z- is -NH-, -O-, -S-, or -CH₂-;
R¹ is selected from hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, alkenyl, alkynyl, cyano, formyl, or alkoxycarbonyl; and
R², R³, R⁴, R⁵, R⁸, and R⁹ are each independently hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, phosphocarbonyl, substituted silyl, alkenyl, or alkynyl.

2. A compound of a formula (I), or a stereoisomer, tautomer, or pharmaceutically acceptable salt thereof, 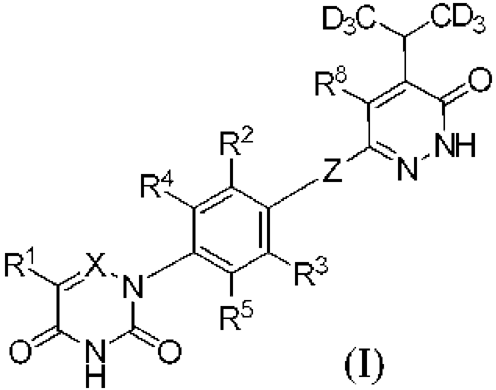
wherein X is N or C;
-Z- is -NH-, -O-, -S-, or -CH₂-;
R¹ is selected from hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, alkenyl, alkynyl, cyano, formyl, or alkoxycarbonyl; and
R², R³, R⁴, R⁵, and R⁸ are each independently hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, phosphocarbonyl, substituted silyl, alkenyl, or alkynyl.

3. A compound of a formula (II), or a stereoisomer, tautomer, or pharmaceutically acceptable salt thereof, 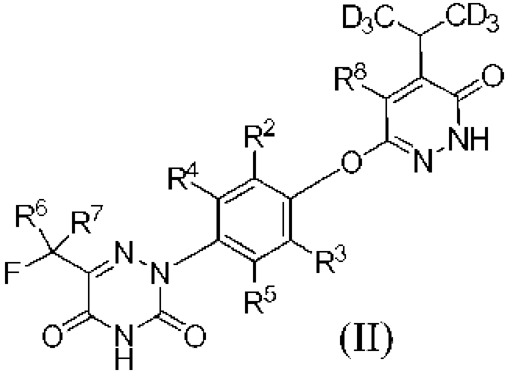 wherein R², R³, R⁴, R⁵, R⁶, R⁷, and R⁸ are each independently hydrogen, deuterium, halogen, alkyl, deuteroalkyl, haloalkyl, cycloalkyl, heterocyclyl, heterocyclylalkyl, aryl, heteroaryl, hydroxyl, amino, sulfonyl, phosphocarbonyl, substituted silyl, alkenyl, or alkynyl.

4. A compound of any one of formulae 1 to 12 and formulae 16 to 18 below, or a stereoisomer, tautomer, or pharmaceutically acceptable salt thereof,

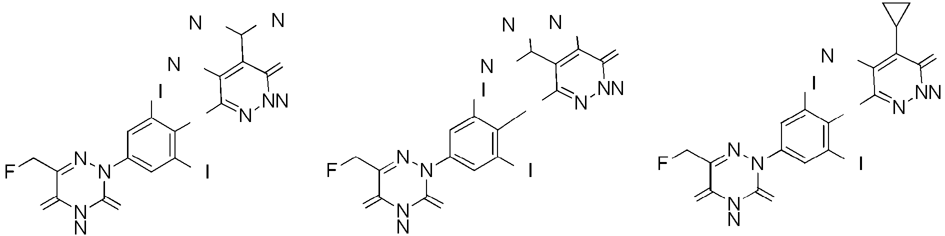

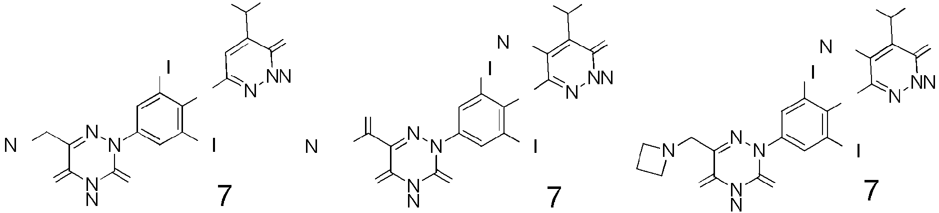

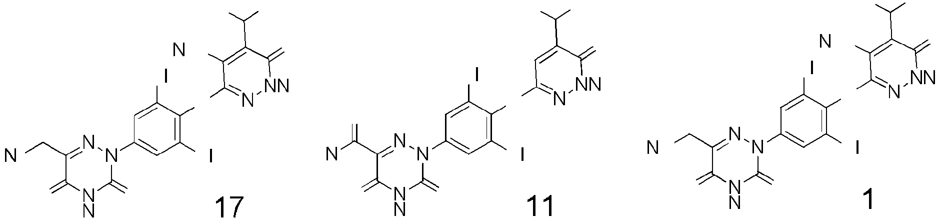

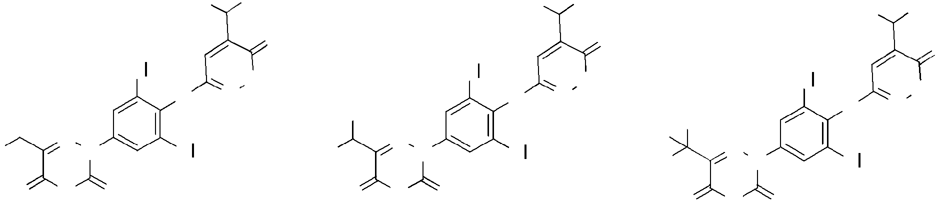

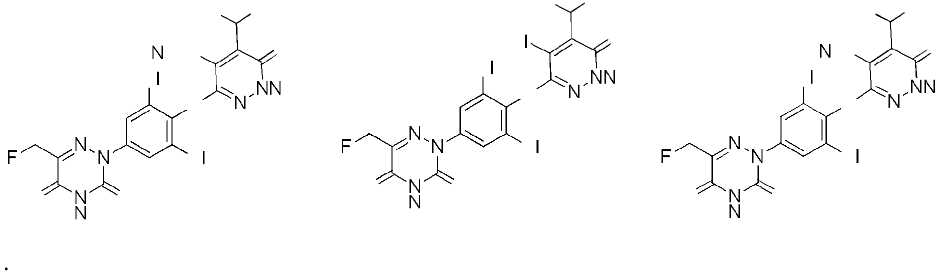

5. A pharmaceutical composition, comprising the compound, or the stereoisomer, tautomer or pharmaceutically acceptable salt thereof according to any one of claims 1 to 4, and a pharmaceutically acceptable carrier.

6. Use of the compound, or the stereoisomer, tautomer or pharmaceutically acceptable salt thereof according to any one of claims 1 to 4, or the pharmaceutical composition according to claim 5 in the manufacture of a thyroid hormone receptor (THR) agonist.

7. The use according to claim 6, wherein a disease that can be treated by the THR agonist is a metabolic disease, preferably obesity, hyperlipidemia, hypercholesterolemia, diabetes, non-alcoholic steatohepatitis (NASH), atherosclerosis, cardiovascular disease, hypothyroidism or thyroid cancer.

8. A method for treating a disease with a thyroid hormone receptor (THR) agonist, comprising administering to a subject in need thereof a therapeutically effective amount of the compound, or the stereoisomer, tautomer or pharmaceutically acceptable salt thereof according to any one of claims 1 to 4.

9. The method according to claim 8, wherein the disease is a metabolic disease, preferably obesity, hyperlipidemia, hypercholesterolemia, diabetes, non-alcoholic steatohepatitis (NASH), atherosclerosis, cardiovascular disease, hypothyroidism or thyroid cancer.

10. A method for preparing the compound according to any one of claims 1 to 4, comprising the following reaction scheme: 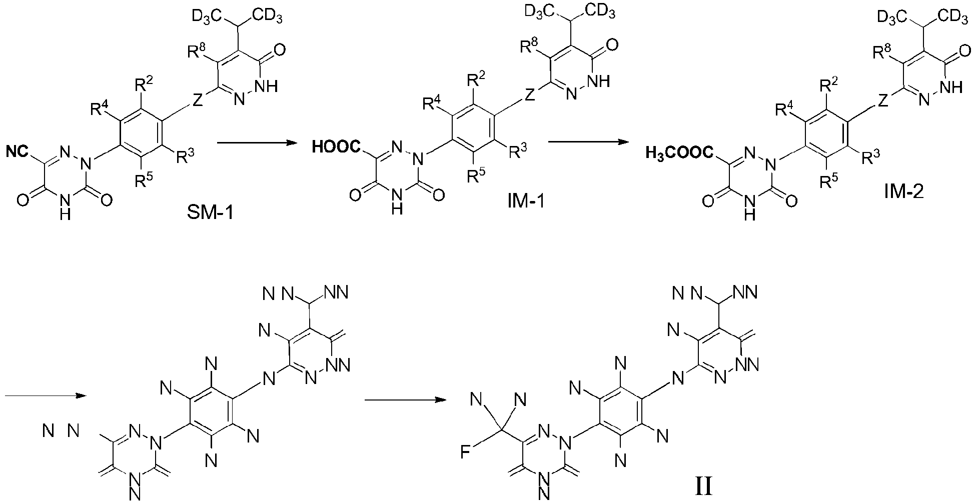 and specifically comprising performing cyano hydrolysis by using SM-1 as a starting material to obtain a compound (IM-1), carrying out an esterification reaction to obtain a compound (IM-2), performing reduction to obtain a compound (IM-3), and carrying out a functional group conversion reaction to obtain a compound (II), wherein R², R³, R⁴, R⁵, R⁶, R⁷, and R⁸ are as defined in claim 3.
